# EUROPEAN PATENT APPLICATION

(11) **EP 2 072 037 A1**
(43) Date of publication of application: **24.06.2009**
(21) Application number: 07425795.7
(22) Date of filing: 17.12.2007
(51) Int. Cl.: A61K 8/41, A61K 8/44, A61Q 5/10

(54) **Hair dyeing compositions**

(71) Applicant: Farmen International Cosmetics Distribution S.p.A, 10036 Settimo Torinese (TO) (IT)
(72) Inventor: Manzetti, Giovanni, 10036 Settimo Torinese (Torino) (IT); Gazzaniga, Giancarlo, 27056 Salice Terme (Pavia) (IT)
(74) Representative: Freyria Fava, Cristina

(57) **Abstract**

Oxidative hair dyeing composition comprising paratoluenediamine or paraphenyldiamine as dyestuff and pyroglutamic acid as substance capable of reducing skin irritation and sensitisation, paratoluenediamine or paraphenyldiamine being salified with pyroglutamic acid.

## Description

The present invention concerns hair dyeing compositions containing dyestuffs of the oxidative type. In particular, the present invention concerns compositions for the oxidative dyeing of hair that are capable of reducing the irritant and sensitising power of the dye itself or of its individual components.

Oxidative hair dyes, marketed throughout the world for over 70 years, chiefly use two chemical dyes acting *in situ* on the keratin structure of the hair: paraphenyldiamine (2,5PD) and paratoluenediamine (2,5TD) in concentrations that can range from 1% to 2.5% by weight.

Both of these dyes belong to the category of the group III aromatic amines.

The permanent coloration that forms on the hair keratin derives from the reaction of the three principal components of the dye: paratoluenediamine or paraphenyldiamine, coupling agents (such as for example resorcinol and/or metaminophenol) and oxidant agents (normally consisting of hydrogen peroxide). The development substances and the coupling agents are mixed together in a cream base that we may call phase 1. The hydrogen peroxide, in liquid or cream form, comprises phase 2. The two phases are mixed together, after which the composition acquires dyeing capability and penetrates through the scales of the hair, colouring its entire structure and replacing the hair's natural coloration. The colour that is formed is not removed by shampoo.

Although group III aromatic amines have not been recognised as carcinogens, they must be kept under tight control. The International Agency for Cancer Research declared in 2005 that there is no scientific evidence to support the fact that hair dye can cause cancer. There are, however, frequent periodic alarms.

Numerous epidemiological studies, such as that published in 2005 in the prestigious American journal "Personal Use of Hair Dyes and Risk of Cancer", have ruled out any link between the use of hair dyes and breast and bladder cancer. In the few studies with positive results, the evidence linking cause and effect is too weak to claim there is any danger for public health deriving from the use of such dyestuffs.

However, the problem of allergic sensitisation to the substances contained in oxidative hair dyes remains, considering the high percentage of people making repeated use of such dyes (30% of women in Europe, 40% in the United States). This sensitisation may, furthermore, be crossed with that to foodstuffs and drugs.

With the goal of avoiding or limiting these phenomena, European legislation on cosmetics requires that, before using oxidative dyes, the allergic sensitivity of the subject to whom this composition is to be applied must be checked. This is extremely important when the dye contains biologically active molecules such as aromatic diamines. In order to exclude the onset of hypersensitivity or even allergic reactions, a small quantity of product must be applied to the skin (normally behind the ear) the day before the dye is used.

In the past, some studies have been performed aimed at developing hair dyeing compositions with reduced irritant power. For example, from the document DE 297 10 628 U, it is known that oxidative hair dyes can be associated with substances to treat the skin, selected from among proteins, hydrolysed proteins, panthenol, allantoin, pyrrolidoncarboxylic acids and their salts, plant extracts, vitamins and saturated and unsaturated C₁₆₋₂₂, fatty acids, of natural or synthetic origin.

Despite this preamble, the need still exists to produce oxidative hair dyes that are not harmful to the individual, since skin reactions of irritation and sensitisation in such subjects have not effectively been reduced, in particular in subjects at risk, among whom are those with psoriasis and/or allergic reactions and with hyper-sensitisation to the oxidative dyes normally used.

The present patent applicant has thus been active in the development of new hair dyeing compositions that present a lower irritant and sensitising power than the normal hair dyes in current use, in order to reduce the risks linked with their use to a minimum and enable a wider public to use them, including persons to whom, due to health problems, the use of such compositions is not normally available.

This purpose is achieved thanks to the technical solution described in the attached claims. The claims form an integral part of the technical instruction provided here in relation to the invention.

In particular, this purpose is achieved through the development of hair dyeing compositions based on oxidant dyes comprising, as well as the dyestuffs consisting of paratoluenediamine or paraphenyldiamine, at least one substance that is capable of reducing or avoiding the risk of irritation and sensitisation. More specifically, this substance consists of pyroglutamic acid (or pyrolidone carboxylic acid, PCA).

In further detail, the compositions subject of the present invention comprise the dyestuff, paratoluenediamine or paraphenyldiamine, salified with pyroglutamic acid.

In a further embodiment of the present invention, the hair dyeing compositions also contain emollient substances such as C₁₂-C₁₅ alkyl lactate and/or tridecylsalicylate, which serve to further reduce the risk of irritation and sensitisation.

The invention will now be described in detail, with reference to some non-limiting examples of the present invention.

Pyroglutamic acid, also known as Natural Moisturizing Factor (NMF), is formed from the enzymatic cyclisation of glutamic acid, one of the basic aminoacids present in the organism, and is present in the protective hydrolipidic film of the skin in amounts of between 10% and 15%.

The use of a compound that is a constituent of the skin to salify paratoluenediamine or paraphenyldiamine has, surprisingly, enabled the applicant to develop hair dyeing compositions that are particularly effective at reducing irritant and allergic phenomena in persons subjected to the dyeing operation.

As well as reducing the risk of irritation and sensitisation, the use of pyroglutamic acid also gives the hair dyeing compositions a further advantage.

In the form of a salt, in particular the sulphate salt, paratoluenediamine presents problems of solubility when used at concentrations close to its maximum concentration. By salifying paratoluenediamine directly in the form of a base with pyroglutamic acid, a salt is obtained that presents in liquid form, with a much higher solubility.

### Example 1

Tables 1 to 4 give the chemical compositions of four hair dyes according to the present invention.

**Table 1.**

| **Composition A - Brown dye** | |
|---|---|
| COMPONENT | QUANTITY % by weight |
| Cetyl-stearyl alcohol | 15 |
| Oleic alcohol | 2 |
| Oleic acid | 2 |
| Pag40 Castor Oil | 3 |
| Oleic diethanolamide | 3.7 |
| Monoethanolamine | 5.2 |
| Sodium bisulphite | 0.6 |
| Pentasodium pentetate | 1 |
| Ascorbic acid | 0.1 |
| Monoethanolamine lauryl sulphate | 3 |
| 2-amino-4-hydroxyethylamine anisol sulphate | 0.120 |
| P-toluenediamine (25%) | 6 |
| P-aminophenol | 0.120 |
| M-aminophenol | 0.264 |
| Demineralised water to | 100 |

**Table 2.**

| **Composition B - Brown dye** | |
|---|---|
| COMPONENT | QUANTITY % by weight |
| Cetyl-stearyl alcohol | 15 |
| Oleic alcohol | 2 |
| Oleic acid | 2 |
| Pag40 Castor Oil | 3 |
| Oleic diethanolamide | 3.7 |
| Monoethanolamine | 5.2 |
| Sodium bisulphite | 0.6 |
| Pentasodium pentetate | 1 |
| Ascorbic acid | 0.1 |
| Monoethanolamine lauryl sulphate | 3 |
| 2-amino-4-hydroxyethylamine anisol sulphate | 0.120 |
| P-toluenediamine salified with PCA (25%) | 6 |
| P-aminophenol | 0.120 |
| M-aminophenol | 0.264 |
| Demineralised water to | 100 |

**Table 3.**

| **Composition C - Light blond dye** | |
|---|---|
| COMPONENT | QUANTITY % by weight |
| Cetyl-stearyl alcohol | 15 |
| Oleic alcohol | 2 |
| Oleic acid | 2 |
| Pag40 Castor Oil | 3 |
| Oleic diethanolamide | 3.7 |
| Monoethanolamine | 5.2 |
| Sodium bisulphite | 0.6 |
| Pentasodium pentetate | 1 |
| Ascorbic acid | 0.1 |
| Triethanolamine lauryl sulphate | 3 |
| 3-amino-4-hydroxyethylamine anisol sulphate | 0.120 |
| P-phenyldiamine salified with PCA (25%) | 1.20 |
| P-aminophenol | 0.120 |
| M-aminophenol | 0.264 |
| Demineralised water to | 100 |

**Table 4.**

| **Composition D - Light blond dye** | |
|---|---|
| COMPONENT | QUANTITY % by weight |
| Cetyl-stearyl alcohol | 15 |
| Oleic alcohol | 2 |
| Oleic acid | 2 |
| Pag40 Castor Oil | 3 |
| Oleic diethanolamide | 3.7 |
| Monoethanolamine | 5.2 |
| Sodium bisulphite | 0.6 |
| Pentasodium pentetate | 1 |
| Ascorbic acid | 0.1 |
| Triethanolamine lauryl sulphate | 3 |
| 3-amino-4-hydroxyethylamino anisol- | 0.120 |
| sulphate | |
| P-phenyldiamine (25%) | 1.20 |
| P-aminophenol | 0.120 |
| M-aminophenol | 0.264 |
| Demineralised water to | 100 |

### Example 2

The procedure to salify paratoluenediamine (2,5TD) and paraphenyldiamine (2,5PD) with pyroglutamic acid (PCA) will now be described.

2,5TD is marketed as the sulphate; 2,5TD sulphate (100 g) is dissolved in water (500 g), basified with caustic soda (30 g) with separation of sodium sulphate and formation of 2,5TD free base. The free base is distilled and purified, diluted at 25% with water and then salified with PCA at the stoichiometric ratio or with a slight excess of PCA (30 g per 100 g of 2,5TD base at 25%).

As a salt, in particular as the sulphate, paratoluenediamine has poor solubility when close to the maximum allowed concentration. Salification of paratoluenediamine with pyroglutamic acid, however, markedly increases its solubility.

2,5PD is salified as a free base, diluted at 25% with water, at the stoichiometric ratio or with a slight excess of PCA (30 g per 100 g of 2,5PD at 25%).

### Example 3

The applicant initially performed some experiments to check that hair dyeing formulations based on paratoluenediamine (2,5 TD) in association with pyroglutamic acid (PCA) do not possess a reduced colorant power than paratoluenediamine used alone.

The experiment was performed using formulations not containing ammonia in the presence of PCA and paratoluenediamine base (2,5TD at 25%) in the formation of colour on the hair.

The test gave positive results: no interference of PCA with the dyestuff was revealed, rather the colour was found to be of increased brightness.

### Example 4

The applicant verified that the use of paratoluenediamine salified with pyroglutamic acid (PCA) was not toxic and that it enables skin irritation and sensitisation to be greatly reduced.

To evaluate the pro-sensitising potential of compositions A and B, illustrated in Example 1, two in vitro tests were used: a first evaluation of its cytotoxicity on human monocytes through MTT and a subsequent evaluation of the pro-sensitising stimulus on these cells through FACS.

In the test performed, it was deemed appropriate to use a cell model that is suited to evaluate immunological reactivity, that is immune cells, which were exposed to prolonged contact (48 h) with composition A or composition B at two different concentrations.

More precisely, the test in question was done on the cell line known as THP-1 (monocytoid cell line), this type of cell being primarily involved in the immune response of the skin.

On the THP-1 cells, modulation of the expression of two co-stimulatory molecules, CD80 (B7.1) and CD86 (B7.2), was evaluated taking as positive control a typical contact sensitising substance, nickel sulphate, which is capable of stimulating allergising reactions including *in vivo* and is widely used in *in vitro* tests.

CD80 (B7.1) and CD86 (B7.2) are two glycoproteins that act as co-stimulants to activate T lymphocytes during the immune response: once the monocyte has processed the antigen and exposed it on its plasma membrane, so that it will be recognised by the lymphocytes, further expression of CD80 (B7.1) and CD86 (B7.2) permits its contact with the CD28 receptors situated on the lymphocyte surface, modulating proliferation and differentiation of the lymphocytes.

The human monocytoid cell line THP-1 was cultured in RPMI containing 10% FCS and 2% glutamine.

To avoid alteration of the results due to cell death, the specimens corresponding to composition A and composition B were previously mixed with the oxidant cream (comprising 125 volume hydrogen peroxide (16% by weight) sodium stammate (0.020% by weight), glycerine (0.500% by weight), stearylic alcohol 20 OE (3.5% by weight), phosphonic acid (0.300% by weight), demineralised water (to 100% by weight)) in the proportion of 1:1.5, left to act for 15 minutes, then dissolved in ethanol and diluted in culture medium at different concentrations.

On the cells thus treated, the MTT test was then performed (Mossman, J. Immunol. Methods 1993; 65: 55-63) to evaluate the cytotoxic effect. The data obtained show that, unlike SLS (sodium lauryl sulphate - positive control), the combination of 2,5TD with PCA has no cytotoxic effect on human keratinocytes, independently of the presence of emollient substances. This result should be considered highly predictive of a lack of irritant effects *in vivo.*

Subsequently, compositions A and B were used to determine their pro-sensitising potential.

For this purpose, dilutions of compositions A and B with the oxidant cream were added to the culture medium and the cells were cultured for 48 hours at 37 °C at 5% CO₂. After exposure, the cells were subjected to Tripan Blue staining and observed under the microscope to determine their vitality. They were then washed and marked with antibodies directed against B7.1 and B7.2 and subjected to flow cytometric analysis (FACS) to evaluate the MFI (mean fluorescence intensity), which is proportional to the number of marked molecules per cell, and is thus representative of their level of expression. Other morphological qualitative parameters linked to cell necrosis and apoptosis, connected to the process of allergic excitation, were also evaluated.

The results of these tests are reported in Table 5.

**Table 5.**

| **Specimen** | **CD80 (MFI*)** | **CD86 (MFI*)** |
|---|---|---|
| Nickel sulphate 20 ug/mL | 39.44 | 91.78 |
| Nickel sulphate 10 ug/mL | 4.56 | 30.79 |
| Nickel sulphate 4 ug/mL | 1.01 | 0.17 |
| Composition A 0.07 mg/mL | -0.11 | 0.01 |
| Composition A 0.0014 mg/mL | -0.1 | -0.03 |
| Composition B 0.07 mg/mL | -0.14 | -0.24 |
| Composition B 0.0014 mg/mL | -0.22 | -0.44 |

| | | |
|---|---|---|
| * Mean Fluorescence Intensity: geometric mean of the intensity of fluorescence of the cells fluorescing with the antibody used. | | |

The results obtained show that, unlike nickel sulphate, the combination of 2,5TD with PCA (composition B) does not increase expression of any of the markers investigated in human monocytes, showing that it does not possess any potential to stimulate the immune system mediated by the monocyte/macrophage system, thus it has no allergising potential.

### Example 5

To evaluate the skin irritation potential of compositions C and D (containing paraphenyldiamine) illustrated in example 1, an *in vitro* test was used, consisting of a cytotoxicity assay performed on a line of human keratinocytes (transformed human keratinocytes, HaCaT cells) that maintain full epidermal differentiation capability.

It should be remembered that cytotoxicity tests on fibroblasts and keratinocytes in monolayer are currently used as predictive indicators of the irritant potential of products for topical use, cosmetics or individual compounds, to replace the use of animals, as established by the regulations in force in the sector.

In this case, the goal of the test was to determine the effects of compositions C and D illustrated in Example 1 on the proliferation of keratinocytes through the cell vitality test, indirectly measured by MTT staining (Mossnam T, 1993, J. Immunol. Methods, 65:55-63).

The specimen comprising composition C or D was previously mixed with oxidant cream in the proportion 1:1.5 and left to act for 15 minutes.

The specimen was then dissolved directly in the cell culture medium enriched with 10% FCS, in 6 different final dilutions between 5 and 15 mg/l.

The cells were treated for two hours to simulate the conditions of use of the dye. At the end of the two hours, the cells were subjected to the MTT test.

From the cytotoxicity data displayed in graph form versus the concentration of the product tested, a dose-response curve may be drawn, from which the theoretical regression curve and the theoretical value of IC50, corresponding to the concentration that induces a reduction in cell vitality of 50% compared to untreated cells, can be determined. From this value, the irritant potential of composition C and composition D can be estimated. Negative control comprised no treatment, whereas the surfactant sodium lauryl sulphate (SLS), whose cytotoxicity is well known, was used as positive control. Each experiment was done in triplicate, on cells plated 24 hours previously.

The test results are shown in tables 6-8.

**Table 6.**

| **SLS (positive control)** | | | | | |
|---|---|---|---|---|---|
| Dose mg/mL | 0.5 | 0.25 | 0.12 | 0.06 | 0.03 |
| Cell vitality % (versus negative control) | 3.1 | 3.4 | 74.0 | 109.7 | 101.5 |
| Standard deviation | 0.9 | 0.3 | 4.1 | 4.3 | 4.0 |
| **IC₅₀ = 0.148 mg/mL** | | | | | |

**Table 7.**

| **Composition C** | | | | | | |
|---|---|---|---|---|---|---|
| Dose mg/mL | 5 | 2.5 | 1.25 | 0.6 | 0.3 | 0.15 |
| Cell vitality % (versus negative control) | 62.8 | 73.6 | 85.5 | 104.7 | 116.0 | 115.5 |
| Standard deviation | 4.7 | 7.5 | 4.9 | 12.0 | 19.9 | 12.5 |
| **IC₅₀ > 5 mg/mL** | | | | | | |

**Table 8.**

| **Composition D** | | | | | | |
|---|---|---|---|---|---|---|
| Dose mg/mL | 5 | 2.5 | 1.25 | 0.6 | 0.3 | 0.15 |
| Cell vitality % (versus negative control) | 39.1 | 50.9 | 81.7 | 108.2 | 109.5 | 104.4 |
| Standard deviation | 2.4 | 3.0 | 1.4 | 5.3 | 5.4 | 4.9 |
| **IC₅₀ = 3.26 mg/mL** | | | | | | |

The results obtained show that, unlike SLS, the combination of 2,5PD with PCA has no cytotoxic effect on human keratinocytes, independently of the presence of emollient substances. This result may be considered highly predictive of the lack of irritant effects *in vivo.*

### Example 6

In the light of these results, the subsequent phase of testing comprised studies to determine the allergenic and hypersensitising potential of compositions A, B, C and D on volunteers, previously sensitised to paratoluenediamine and to paraphenyldiamine.

Also in this case, the application of a dye produced with paratoluenediamine or with paraphenyldiamine salified with pyroglutamic acid produced no irritation and/or none of the usual allergic reactions in any subject, confirming the validity of the associations PCA/2,5TD and PCA/2,5PD.

To evaluate and confirm the characteristics of the composition according to the present invention, daily applications of these dyeing compositions have been continued for eight months on volunteers, including some subjects suffering from psoriasis. In approximate terms, at least 200 applications on volunteers have been made, repeating applications on the same subject for approximately 6/7 times.

In none of these applications has any skin irritation or allergy occurred. In parallel, tests have been done at the Dermatological Clinic of the University of Siena, in which 50 patch-tests have been applied to volunteers, without any type of skin problem being reported.

### Example 7

In order to further improve the hair dyeing composition based on paratoluenediamine, the applicant carried out further investigations on the use of other emollient substances and, precisely, C₁₂₋₁₅ alkyl lactate and/or tridecyl salicylate.

The functional characteristics contributed by the above two ingredients to the hair dyeing compositions according to the present invention are as follows:
- C₁₂₋₁₅ alkyl lactate is a substance that leaves the hair soft, untangled and shiny, as well as increasing the mixture's anti-irritant power;
- tridecylsalicylate gives a protective action against the sun's rays, in particular UV rays.

It was found that, in the subjects to whom a hair dyeing composition according to the present invention had been applied, the hair colour was preserved for a long period of time and did not fade, indicating an excellent resistance to washing.

In this case too the subjects tested in the *in vivo* evaluation gave a positive outcome, since there were no reports of irritation or allergic reactions.

Naturally, production details and embodiments may be widely varied with regard to what is described and illustrated without thereby departing from the sphere of protection of the present invention, as defined by the attached claims.

## Claims

1. Hair dyeing composition comprising at least one dyestuff of the oxidative type and at least one substance capable of reducing skin irritation and sensitisation, in which said at least one oxidative agent is selected from between paratoluenediamine and paraphenyldiamine, said at least on substance capable of reducing irritation and sensitisation consists of pyroglutamic acid, **characterised in that** said dyestuff is present as a salt of pyroglutamic acid.

2. Composition according to claim 1, further including C₁₂₋₁₅ alkyl lactate.

3. Composition according to claim 1 or claim 2, further including tridecyl salicylate.

4. Composition according to any of the above claims, further including monoethanolamine.

5. Composition according to any of the above claims, in which the dyestuff:pyroglutamic acid ratio in the salt is a stoichiometric ratio.

6. Composition according to any of the above claims, in which the paratoluenediamine:pyroglutamic acid ratio in the salt is a ratio between 1:1 and 1:3.

7. Composition according to any of the claims from 1 to 5, in which the paraphenyldiamine:pyroglutamic acid ratio in the salt is a ratio between 1:1 and 1:6.

8. Composition according to any of the above claims, in which the paratoluenediamine pyroglutamate salt is present in a quantity, expressed as % by weight, between 0.01 and 5, preferably between 0.1 and 2.

9. Composition according to any of the above claims, in which the paraphenyldiamine pyroglutamate salt is present in a quantity, expressed as % by weight, between 0.01 and 4, preferably between 0.01 and 2.

10. Composition according to any of the claims from 2 to 9, in which C₁₂₋₁₅ alkyl lactate is present in a quantity, expressed as % by weight, between 1 and 3, preferably between 1 and 1.5.

11. Composition according to any of the claims from 3 to 10, in which tridecyl salicylate is present in a quantity, expressed as % by weight, between 1 and 3, preferably between 1 and 1.5.

12. Composition according to any of the claims from 4 to 11, in which monoethanolamine is present in a quantity, expressed as % by weight, between 4 and 8, preferably between 5 and 6.
